(19) Europäisches Patentamt
European Patent Office
Office européen des brevets

(11) Publication number : 0 203 736 B1

# (12) EUROPEAN PATENT SPECIFICATION

(45) Date of publication of patent specification : 23.11.89

(51) Int. Cl.$^4$ : C 07 D473/32

(21) Application number : 86303337.9

(22) Date of filing : 01.05.86

## (54) Antiviral compounds.

(30) Priority : 02.05.85 GB 8511223
02.05.85 GB 8511225

(43) Date of publication of application : 03.12.86 Bulletin 86/49

(45) Publication of the grant of the patent : 23.11.89 Bulletin 89/47

(84) Designated contracting states : AT BE CH DE FR GB IT LI LU NL SE

(56) References cited :
EP-A- 0 108 285
GB-A- 1 523 865
GB-A- 2 104 070
The file contains technical information submitted after the application was filed and not included in this specification

(73) Proprietor : THE WELLCOME FOUNDATION LIMITED
183-193 Euston Road
London NW1 2BP (GB)

(72) Inventor : Beauchamp, Lilla Marie
3007 Wycliff Road
Raleigh North Carolina 27607 (US)
Inventor : Krenitsky, Thomas Anthony
106 Laurel Hill Road
Chapel Hill North Carolina 27514 (US)

(74) Representative : Garrett, Michael et al
The Wellcome Foundation Limited Group Patents and Agreements Langley Court
Beckenham Kent BR3 3BS (GB)

EP 0 203 736 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

## Description

The present invention relates to novel purine derivatives having valuable antiviral activity particularly against viruses of the herpes family.

U.K. Patent Specification No. 1523865 describes a broad class of purine derivatives containing an acyclic side chain in the 9-position. These purine derivatives have been found to have antiviral activity against various classes of DNA viruses particularly against herpes viruses such as herpes simplex. Among these derivatives, 9-(2-hydroxyethoxymethyl) guanine (otherwise known as acyclovir) has been found to have particularly good activity against herpes simplex virus. Other purine derivatives containing an acyclic side chain in the 9-position have also been described including for example analogues of acyclovir containing a 2-hydroxy-1-hydroxymethylethoxymethyl group in the 9-position, described in UK Patent Specification No. 2104070A and acyclovir analogues which are unsubstituted at the 6-position, as described in UK Patent Specification No. 2130204A.

We have now discovered advantageous antiviral activity in a new class of 6-H analogues of acyclovir as described below.

According to the present invention we provide a compound of the formula.

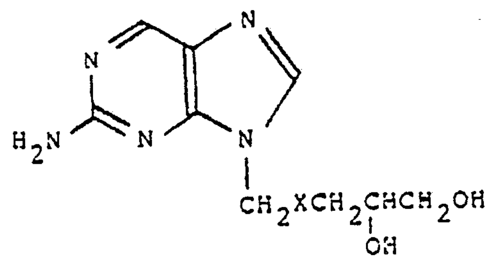

(I)

(wherein X is oxygen or sulphur and physiologically acceptable salts and esters thereof.

Salts of the compound of formula (I) which may be conveniently used in therapy include physiologically acceptable salts of organic acids as lactic, acetic, malic or p-toluenesulphonic acid as well as physiologically acceptable salts of mineral acids such as hydrochloric or sulphuric acid.

Esters of the compound of formula (I) which may be conveniently used in therapy include those containing a formyloxy or $C_{1-16}$ (for example $C_{1-16}$) branched or straight chain alkanoyloxy (e. g. acetoxy or propionyloxy), optionally substituted aralkanoyloxy (e. g. phenyl-$C_{1-4}$ alkanoyloxy such as phenyl-acetoxy) or optionally substituted aroyloxy (e. g. benzoyloxy or naphthoyloxy) or phosphate ester grouping at one or both of the terminal positions of the 9-side chain of the compounds of formula (I). The above-mentioned aralkanoyloxy and aroyloxy ester groups may be substituted, for example by one or more halogen (e. g. chlorine or bromine) atoms or amino, nitrile or sulphamido groups, the aryl moiety of the grouping advantageously containing 6 to 10 carbon atoms.

According to a further feature of the present invention we provide a compound of formula (I) and physiologically acceptable salts and esters thereof for use in therapy for example in the treatment or prophylaxis of a viral disease in an animal, e. g. a mammal such as man.

The present invention also provides compounds for the treatment or prophylaxis of a viral disease in an animal, e. g. a mammal such as man which comprises administering to the animal an effective antiviral amount of the compound of formula (I) or a physiologically acceptable salt or ester thereof.

The present invention further provides the use of a compound of formula (I) or a physiologically acceptable salt or ester thereof in the manufacture of a medicament for the treatment or prophylaxis of a viral disease.

The compounds of formula (I) and their physiologically acceptable salts and esters have a particularly effective activity in vivo against DNA viruses particularly those of the herpes family which includes herpes simplex, varicella zoster, cytomegalovirus and Epstein-Barr virus, as well as hepatitis B.

The compound of formula (I) and the physiologically acceptable salts and esters thereof (hereafter collectively referred to as the active ingredients) may be administered by any route appropriate to the condition to be treated suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with for example the condition of the recipient.

For each of the above-indicated utilities and indications the amount required of an active ingredient (as above defined) will depend upon a number of factors including the severity of the condition to be treated and the identity of the recipient and will ultimately be at the discretion of the attendant physician or veterinarian. In general however, for each of these utilities and indications, a suitable, effective dose will be in the range 0.1 to 250 mg per kilogram bodyweight of recipient per day, preferably in the range 1 to 100 mg per kilogram bodyweight per day and most preferably in the range 5 to 20 mg per kilogram bodyweight per day; an optimum dose is about 10 mg per kilogram bodyweight per day. (Unless

otherwise indicated all weights of active ingredient are calculated as the parent compound of formula (I) : for salts and esters thereof the figures would be increased proportionately.) The desired dose is preferably presented as two, three, four or more sub-doses administered at appropriate intervals throughout the day. These sub-doses may be administered in unit dosage forms, for example, containing 10 to 1 000 mg, preferably 20 to 500 mg and most preferably 100 to 400 mg of active ingredient per unit dosage form.

While it is possible for the active ingredients to be administered alone it is preferable to present them as pharmaceutical formulations. The formulations, both for veterinary and for human use, of the present invention comprise at least one active ingredient, as above defined, together with one or more acceptable carriers therefor and optionally other therapeutic ingredients. The carrier(s) must be « acceptable » in the sense of being compatible with the other ingredients of the formulation and not deleterious to the recipient thereof.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient ; as a powder or granules ; as a solution or a suspension in an aqueous liquid or a non-aqueous liquid ; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For infections of the eye or other external tissues e. g. mouth and skin, the formulations are preferably applied as a topical ointment or cream containing the active ingredient in an amount of, for example, 0.075 to 20 % w/w, preferably 0.2 to 15 % w/w and most preferably 0.5 to 10 % w/w. Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20 %, advantageously 0.5 to 10 % particularly about 1.5 % w/w.

Formulations suitable for topical administration in the mouth include lozenges comprising the active ingredient in a flavoured basis, usually sucrose and acacia or tragacanth ; pastilles comprising the active ingredient in an inert basis such as gelatin and glycerin, or sucrose and acacia ; and mouthwashes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 microns which is administered in the manner in which snuff is taken, i. e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient ; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described. Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type

of formulation in question, for example those suitable for oral administration may include flavouring agents.

The compounds of formula (I) and its their physiologically acceptable salts and esters may be prepared in conventional manner by analogous processes for preparing compounds of similar structure, such as those methods described in U.K. Patent Specification Nos. 1523865, 2104070 and 2130204.

According to a further feature of the present invention there is provided a process for preparing the compounds of formula (I) and physiologically acceptable salts and esters thereof, characterised in that :

a) one deblocks a compound of formula

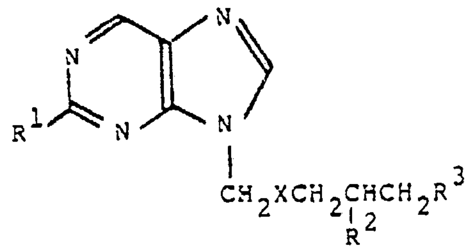

(II)

(wherein X is as hereinbefore defined, $R^1$ represents an amino or blocked amino group, $R_2$ and $R_3$ which may be the same or different, each represents a hydroxy or blocked hydroxy group, providing at least one of $R_1$, $R_2$ and $R_3$ represents a blocked group) or a salt or ester thereof ;

b) one converts a compound of formula

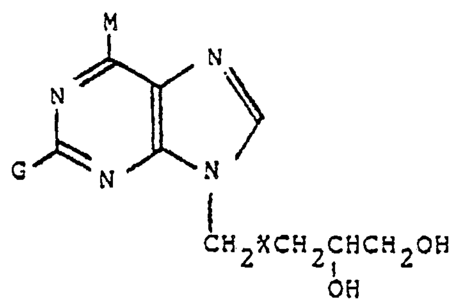

(III)

(wherein X is as defined above and M represents a hydrogen atom or a radical convertible to a hydrogen atom and G represents a radical convertible to an amino group or (when M represents a radical as defined above) an amino group) or a salt or ester thereof, into the compound of formula (I) or a salt or ester thereof ;

c) reacting a compound of formula

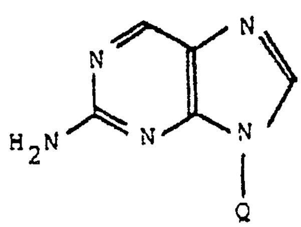

(IV)

(wherein Q represents a leaving group or atom) with a compound of formula

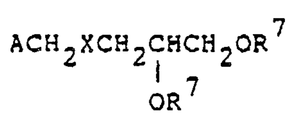

(V)

(wherein X is as defined above, $R^7$ is a blocking group and A represents a leaving group or atom) ;

d) closing a ring in a precursor compound having either the pyrimidine or imidazole ring incompletely formed ; and optionally effecting one or more of the following conversions, in any desired sequence :

i) where the resulting product is a base, converting the said base into a physiologically acceptable acid addition salt thereof ;

ii) where the resulting product is an acid addition salt, converting the said salt into the parent base ;

iii) where the resulting product is the compound of formula (I) or a salt thereof converting the said compound or salt thereof into a physiologically acceptable ester of the said compound or salt ; and

iv) where the resulting product is an ester of the compound of formula (I), converting the said ester into the parent compound of formula (I), a physiologically acceptable ester thereof.

In methods a) and c) the blocking groups may be selected, as appropriate, for example from acyl groups such as $C_{1-4}$ alkanoyl groups e. g. acetyl or pivaloyl, or aroyl groups, e. g. benzoyl ; arylmethyl groups e. g. benzyl ; or tri-$C_{1-4}$ alkylsilyl e. g. trimethysilyl. Arylmethyl blocking groups, may be removed for example by hydrogenolysis, e. g. by hydrogenation in the presence of Raney Nickel or palladium catalyst or by use of sodium in liquid ammonia. Acyl blocking groups may be removed for example by hydrolysis using for example an amine such as methylamine or triethylamine, advantageously in an aqueous medium. Trialkylsilyl blocking groups may be removed for example by solvolysis e. g. with alcoholic or aqueous ammonia, or by alcoholysis.

Conversion of a compound of formula (III) into a compound of formula (I), by method b), can be achieved by various conventional means. For example G may represent an azide group which can be reduced to an amino group by catalytic hydrogenation using a suitable catalyst such as palladium. Alternatively, G may represent a halogen, e. g. chlorine, atom or a mercapto or thio (S =) group which can be converted into a hydrogen atom in a conventional manner, e. g. by catalytic hydrogenation using for example a palladium-charcoal catalyst.

These processes together with other conventional processes are described in Fused Pyrimidine, Part II, Purines Ed. by D. J. Brown (1971), Willey-Intrescience.

In process (c), the group Q in formula (IV) may for example represent a hydrogen atom ; an acyl group, e. g. a $C_{1-4}$ alkanoyl group such as an acetyl group or an aroyl group such as a benzoyl group ; or a tri-$C_{1-4}$ alkylsilyl group such as a trimethylsilyl group. The group A in formula (V) may for example represent a halogen atom (e. g. chlorine) or an acyloxy group wherein the acyl moiety may be for example a $C_{1-4}$ alkanoyl group such as acetyl, or an aroyl group such as benzoyl. The reaction may be conveniently effected in a strong polar such as dimethylformamide or hexamethylphosphoramide, advantageously in the presence of a base such as triethylamine or potassium carbonate. Alternatively, a thermal condensation may be effected by heating the compounds of formulae (IV) and (V) in the presence of a catalytic amount of a strong acid, e. g. sulphuric acid.

The preparation of the physiologically acceptable salts and esters of the compound of formula (I) may be effected in conventional manner, e. g. by appropriate esterification or salt-formation of the compound of formula (I). The optional conversions i)-iv) may also be carried out in conventional manner.

The starting materials employed in the processes described above may also be prepared in conventional manner, e. g. in accordance with the processes described in the above-mentioned UK Patent Specifications.

The following Examples illustrate the present invention.

## Example 1

3-[(2-Amino 9H-purin-9-yl) methoxy-]-1,2-propanediol

a) 3-[2-Amino-6-chloro-9H-purin-9-yl) methoxy]-1,2-diacetoxypropane

A mixture of 5.46 g (32.2 mMO of 2-amino-6-chloropurine, 3.29 g (24.9 mM) of ammonium sulfate, and 100 ml of hexamethyldisilazane was refluxed for 4 hours and evaporated in vacuo to near dryness, then dissolved into about 200 ml of dry toluene. To the mixture was added 9.18 g (32.2 mM) of 3-0-(bromomethyl-1,2-diacetoxy) propanol and the solution was refluxed for 18 hours. The pH 1 mixture was neutralized using triethylamine followed by the addition of 95 % ethanol, then heated for about 20 minutes and evaporated to near dryness. The mixture was partitioned between methylene chloride and water and the organic layer dried over $MgSO_4$, filtered and evaporated in vacuo to an amber oil which was purified by flash chromatography on silica gel eluting with 100 % $CH_2Cl_2$ and yielded 5.974 g (52 %) of the alkylated 2-amino-6-chloropurine intermediate.

b) 3-[(2-Amino-9H-purin-9-yl) methoxy]-1,2-diacetoxypropane

A mixture of 4.215 g (11.8 mM) of 3-[(2-amino-6-chloro-9H-purin-9-yl) methoxy]-1,2-diacetoxypropane, 0.422 g palladium on carbon catalyst. (10 % by weight), 140 ml of 1 : 1 ethanol-tetrahydrofuran, and 3.25 ml triethylamine was shaken at initial pressure of 50 psi $H_2$ on the Parr apparatus for 17 hours. The mixture was filtered and recharged with 0.822 g Pd/C and 2 ml triethylamine under 53 psi $H_2$ and shaken for an additional 19 hours. The celite filtered reaction mixture was evaporated to near dryness and then purified by flash chromatography on silica gel eluting with 50 : 30 : 20 $Et_2O$ : Hexane : MeOH to yield 2.735 g (72 %) of 3-[(2-amino-9H-purin-9-yl) methoxy]-1,2-diacetoxypropane.

c) 3-[(2-Amino-9H-purin-9-yl) methoxy]-1,2-propanediol

2.5 g (7.732 mM) of 3-[(2-amino-9H-purin-9-yl) methoxy]-1,2-diacetoxypropane was stirred at room temperature for ten minutes with 40 ml of aqueous methylamine. The reaction mixture was evaporated to half-volume then extracted twice with methylene chloride to remove acetamide. The $H_2O$ product containing layer was evaporated to an oil which was recrystallised using methanol/acetone to yield 0.426 g (34 %) of the title compound as analytically pure product, mpt. 150-153 °C, (overall yield 13 %).

The following Examples illustrate pharmaceutical formulations according to the invention in which the active compound is a compound of formula (I)

Example 2

| | Tablet |
|---|---|
| Active compound | 200 mg |
| Lactose | 235 mg |
| Starch | 50 mg |
| Polyvinylpyrrolidone | 50 mg |
| Magnesium stearate | 5 mg |
| | 500 mg |

Mix the active compound with the lactose and starch and wet granulate with a solution of the polyvinylpyrrolidone. Dry, sift, blend the granules with magnesium stearate and compress.

Example 3

| | Capsule |
|---|---|
| Active compound | 200 mg |
| Lactose | 184 mg |
| Sodium starch glycollate | 8 mg |
| Polyvinylpyrrolidone | 6 mg |
| Magnesium stearate | 2 mg |

Mix the active compound with the lactose and sodium starch glycollate and wet granulate with a solution of the polyvinylpyrrolidone. Dry, sift, blend the granules with the magnesium stearate and fill into hard gelatin capsules.

Example 4

| | Cream |
|---|---|
| Active compound | 5.00 g |
| Glycerol | 2.00 g |
| Cetostearyl alcohol | 6.75 g |
| Sodium lauryl sulphate | 0.75 g |
| White soft paraffin | 12.50 g |
| Liquid paraffin | 5.00 g |
| Chlorocresol | 0.10 g |
| Purified water | to 100.00 g |

Dissolve the active compound in a mixture of purified water and glycerol and heat to 70 °C. Heat the remaining ingredients together at 70 °C. Add the two parts together and emulsify. Cool and fill into containers.

Example 5

| | Intravenous Injections |
|---|---|
| A) Active compound | 200 mg |
| Sodium hydroxide solution | q. s. to pH 7.0 to 7.5 |
| Water for injections | to 5.0 ml |

Dissolve the active compound in part of the water for injections. Adjust the pH with the sodium hydroxide solution and make up to volume with additional water for injections. Under aseptic conditions, sterilise the solution by filtration, fill into sterile ampoules and seal the ampoules.

| | |
|---|---|
| B) Active compound | 100 mg |
| Sodium hydroxide solution | q. s. to pH 7.0 to 7.5 |
| Mannitol | 125 mg |
| Water for injections | to 2.5 ml |

Dissolve the active compound and mannitol in part of the water for injections. Adjust the pH with the sodium hydroxide solution and make up to volume with additional water for injections. Under aseptic conditions, sterilise the solution by filtration, fill into sterile vials and remove the water by freeze-drying. Seal the vials under an atmosphere of nitrogen and close with a sterile stopper and aluminium collar.

## Antiviral Activity

The compound of Example 1 was tested in vivo to determine its potency in vivo against herpes simplex infections. The compound was tested in comparison with the 6-deoxy analogue 9-(3,4-dihydroxybutyl) guanine (DHBG). The following is description of the testing.

The compounds were tested in CD 1 mice infected with herpes simplex ICI cell culture stock having a titre of $1 \times 10^5$ plaque forming units (pfu)/ml and administered to the mice in a dose of 0.025 ml by the intra-cerebral route.

The test compounds were prepared as aqueous suspensions or solutions, each compound being administered at a dose of 50 mg/kg in a volume of 0.1 ml, by either the oral or sub-cutaneous (s. c.) route, to a treatment group of animals. Each group contained 10 animals for each compound and for each route of administration.

Treatment of the animals commenced 2-3 hours after infection and continued twice daily for 4.5 days.

A group of 13 animals which were infected but untreated served as controls.

Survival times for the test animals were recorded over 14 days and expressed as mean reciprocol survival times (MRST).

The results shown in the following table were obtained in which the figures in the column headed « Survival Times » indicate the number of animals that died on the day indicated in parentheses.

Table

| Compound | Survival Times | MRST |
|---|---|---|
| Example 1 (oral) | 1(6), 1(7), 1(8), 1(10.5) 5 survivors* | 0.059 |
| Example 1 (s.c.) | 3(8), 1(9) 5 survivors* | 0.054 |
| DHBG (oral) | 1(4.5), 3(5), 5(6), 1(7) | 0.18 |
| DHBG (s.c.) | 1(5), 3(6), 5(7), 1(7.5) | 0.155 |
| (Controls) | 6(4), 5(5), 2(6) 0.215 | |

The above results indicate that the compound of Example 1 has a potent antiviral effect in vivo against HSV, particularly in comparison with the 6-hydroxy analogue DHBG.

* 1 mouse died within 24 hours of infection.

**Claims** (for the Contracting States : BE, CH, DE, FR, GB, IT, LI, LU, NL, SE)

1. Compounds of the general formula.

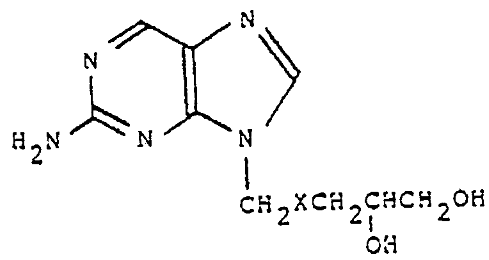

(I)

(wherein X is oxygen or sulphur) and physiologically acceptable salts and esters thereof.

2. Compounds as claimed in claim 1 wherein X is oxygen.

3. 3-[(2-Amino-9H-purin-9-yl) methoxy]-1,2-propanediol.

4. A process for the preparation of compounds of formula (I) (as defined in claim 1) or a physiologically acceptable salt or ester thereof which comprises.

a) deblocking a compound of formula

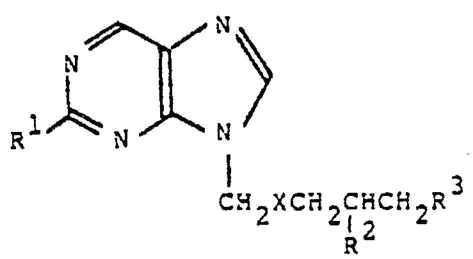

(II)

(wherein X is as defined in claim 1, $R^1$ represents an amino or blocked amino group, $R_2$ and $R_3$ which may be the same or different, each represents a hydroxy or blocked hydroxy group, providing at least one of $R^1$, $R^2$ and $R^3$ represents a blocked group) or a salt or ester thereof ;

b) converting a compound of formula

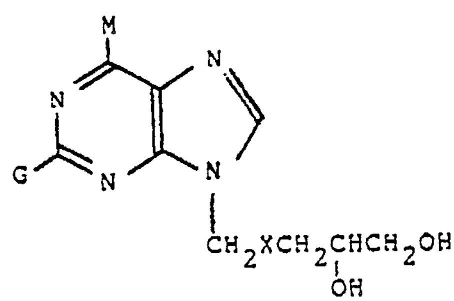

(III)

(wherein X is as defined in claim 1 and M represents a hydrogen atom or a radical convertible to a hydrogen atom and G represents a radical convertible to an amino group or (when M represents a radical as defined above) an amino group) or a salt or ester thereof, into a compound of formula (I) or a salt or ester thereof ;

c) reacting a compound of formula

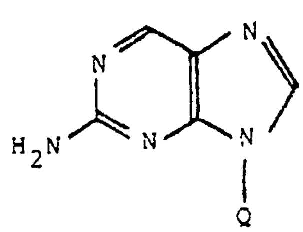

(IV)

(wherein Q represents a leaving group or atom) with a compound of formula

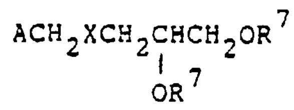

(V)

(wherein X is as defined in claim 1, $R^7$ is a blocking group and A represents a leaving group or atom) ;

d) closing a ring in a precursor compound having either the pyrimidine or imidazole ring incompletely formed ; and optionally effecting one or more of the following conversions, in any desired sequence :

i) where the resulting product is a base, converting the said base into a physiologically acceptable acid addition salt thereof ;

ii) where the resulting product is an acid addition salt, converting the said salt into the parent base ;

iii) where the resulting product is the compound of formula (I) or a salt thereof converting the said compound or salt thereof into a physiologically acceptable ester of the said compound or salt ; and

iv) where the resulting product is an ester of the compound of formula (I), converting the said ester into the parent compound of formula (I), a physiologically acceptable salt thereof or a different physiologically acceptable ester thereof.

5. Pharmaceutical formulations comprising at least one compound as claimed in any of the preceding claims together with at least one pharmaceutical carrier or excipient.

6. A compound of formula (I) (as defined in claim 1) or a physiologically acceptable salt or ester thereof for use in therapy.

7. A compound of formula (I) (as defined in claim 1) or a physiologically acceptable salt or ester thereof for use in the treatment or prophylaxis of viral infections.

8. A compound of formula (I) (as defined in claim 1) or a physiologically acceptable salt or ester thereof for use in the treatment or prophylaxis of herpes simplex infections.

**Claims** (for the Contracting State AT)

1. A process for the preparation of a compound of formula 1.

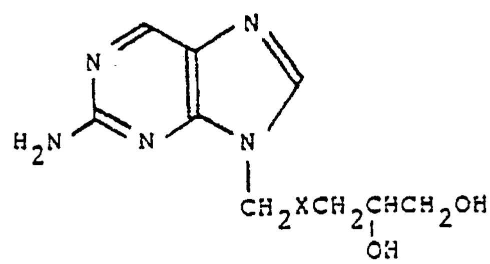

(I)

(wherein X is oxygen or sulphur) or a physiologically acceptable salt or ester thereof, which comprises

a) deblocking a compound of formula

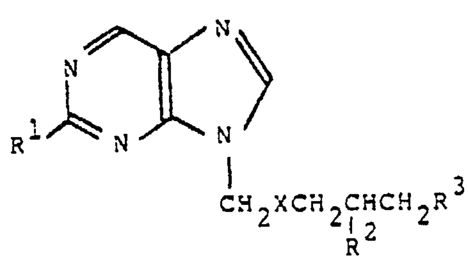

(II)

(wherein X is as defined in claim 1, $R^1$ represents an amino or blocked amino group, $R_2$ and $R_3$ which may be the same or different, each represents a hydroxy or blocked hydroxy group, providing at least one of $R^1$, $R^2$ and $R^3$ represents a blocked group) or a salt or ester thereof ;

b) converting a compound of formula

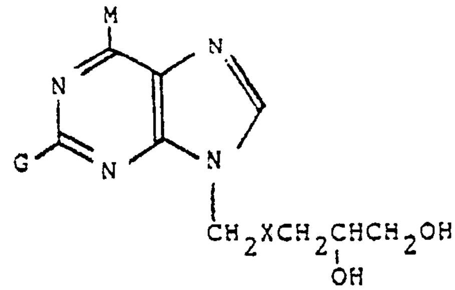

(III)

(wherein X is as defined in claim 1 and M represents a hydrogen atom or a radical convertible to a hydrogen atom and G represents a radical convertible to an amino group or (when M represents a radical as defined above) an amino group) or a salt or ester thereof, into a compound of formula (I) or a salt or ester thereof ;

c) reacting a compound of formula

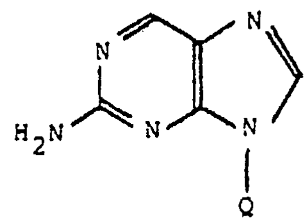

(IV)

(wherein Q represents a leaving group or atom) with a compound of formula

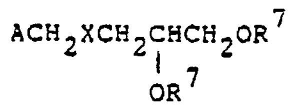

(V)

(wherein n and X is as defined in claim 1, $R^7$ is a blocking group and A represents a leaving group or atom) ;

d) closing a ring in a precursor compound having either the pyrimidine or imidazole ring incompletely formed ; and optionally effecting one or more of the following conversions, in any desired sequence :

i) where the resulting product is a base, converting the said base into a physiologically acceptable acid addition salt thereof ;

ii) where the resulting product is an acid addition salt, converting the said salt into the parent base ;

iii) where the resulting product is the compound of formula (I) or a salt thereof converting the said compound or salt thereof into a physiologically acceptable ester of the said compound or salt ; and

iv) where the resulting product is an ester of the compound of formula (I), converting the said ester into the parent compound of formula (I), a physiologically acceptable salt thereof or a different physiologically acceptable ester thereof.

2. A process for the preparation of compounds as claimed in claim 1 wherein X is oxygen.

3. A process for the preparation of 3-[(2-amino-9H-purin-9-yl) methoxy]-1,2-propanediol as claimed in claim 1.

**Patentansprüche** (für die Vertragsstaaten : BE, CH, DE, FR, GB, IT, LI, LU, NL, SE)

1. Verbindungen der allgemeinen Formel

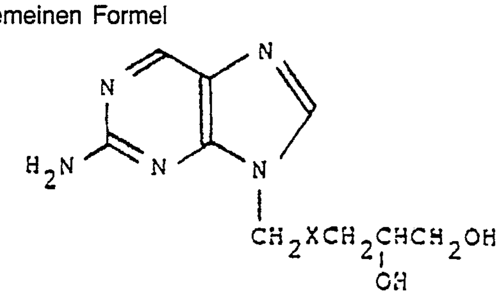

(I)

(in der X Sauerstoff oder Schwefel ist) und deren physiologisch annehmbare Salze und Ester.

2. Verbindungen nach Anspruch 1, in der X Sauerstoff ist.

3. 3-[(2-Amino-9H-purin-9-yl) methoxy]-1,2-propandiol.

4. Verfahren zur Herstellung der Verbindungen der Formel (I) (wie in Anspruch 1 definiert) oder eines physiologisch annehmbaren Salzes oder Esters davon, bei dem man :

a) eine Verbindung der Formel

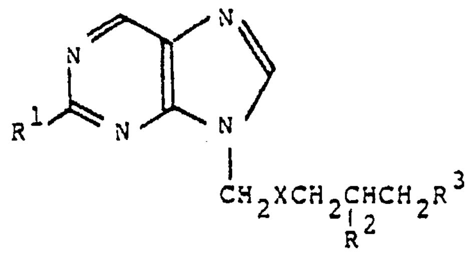

(II)

(in der X die in Anspruch 1 gegebene Bedeutung aufweist, $R^1$ eine Amino- oder blockierte Aminogruppe bedeutet, $R^2$ und $R^3$, die gleich oder verschieden sein können, jeweils eine Hydroxy- oder blockierte Hydroxygruppe bedeuten, mit der Maßgabe, daß mindestens eines von $R^1$, $R^2$ und $R^3$ eine blockierte Gruppe bedeutet, oder ein Salz oder Ester davon, entblockt ;

b) eine Verbindung der Formel

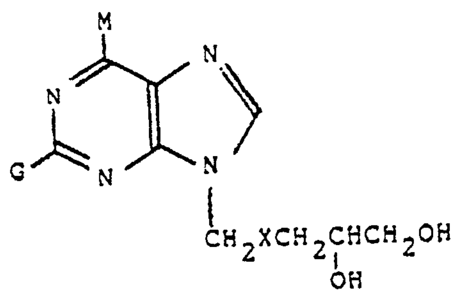

(III)

(in der X die in Anspruch 1 gegebene Bedeutung aufweist und M ein Wasserstoffatom oder einen in ein Wasserstoffatom überführbaren Rest bedeutet und G einen in eine Aminogruppe überführbaren Rest, oder (wenn M einen Rest, wie vorhin definiert, bedeutet) eine Aminogruppe bedeutet, oder ein Salz oder Ester davon in eine Verbindung der Formel (I) oder ein Salz oder Ester davon überführt ;

c) eine Verbindung der Formel

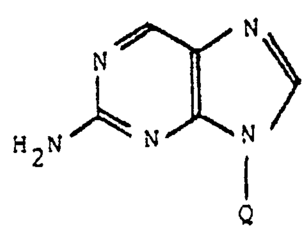

(IV)

(in der Q eine austretende Gruppe oder Atom bedeutet) mit einer Verbindung der Formel

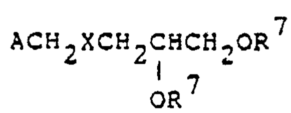

(V)

in der X die in Anspruch 1 gegebene Bedeutung aufweist, $R^7$ eine Blockierungsgruppe ist und A eine austretende Gruppe oder Atom bedeutet, umsetzt ;

d) einen Ring in einer Vorstufenverbindung, in der entweder der Pyrimidin oder der Imidazolring unvollständig gebildet vorliegt, schließt ; und gegebenenfalls eine oder mehrere der folgenden Umwandlungen in jeder gewünschten Reihenfolge durchführt :

i) wenn das erhaltene Produkt ein Base ist, Überführung der Base in ein physiologisch annehmbares Säureadditionssalz ;

ii) wenn das erhaltene Produkt ein Säureadditionssalz ist, Überführung des Salzes in die Stammbase ;

iii) wenn das erhaltene Produkt die Verbindung der Formel (I) oder ein Salz davon ist, Überführung dieser Verbindung oder des Salzes in einen physiologisch annehmbaren Ester dieser Verbindung oder des Salzes, und

iv) wenn das erhaltene Produkt ein Ester der Formel (I) ist, Überführung des Esters in die Stammverbindung (I), ein physiologisch annehmbares Salz davon oder in einen verschiedenen physiologisch annehmbaren Ester.

5. Pharmazeutische Formulierungen enthaltend mindestens eine Verbindung, wie in einem der vorhergehenden Ansprüche beansprucht, zusammen mit mindestens einem pharmazeutischen Trägerstoff oder Verdünnungsmittel.

6. Verbindung der Formel (I) (wie in Anspruch 1 definiert) oder ein physiologisch annehmbares Salz oder Ester davon zur Verwendung in der Therapie.

7. Verbindung der Formel (I) (wie in Anspruch 1 definiert) oder ein physiologisch annehmbares Salz oder Ester davon zur Verwendung in der Behandlung oder Prophylaxe von viralen Infektionen.

8. Verbindung der Formel (I) (wie in Anspruch 1 definiert) oder ein physiologisch annehmbares Salz oder Ester davon zur Verwendung in der Behandlung oder Prophylaxe von Herpes simplex Infektionen.

**Patentansprüche** (für den Vertragsstaat AT)

1. Verfahren zur Herstellung einer Verbindung der Formel I

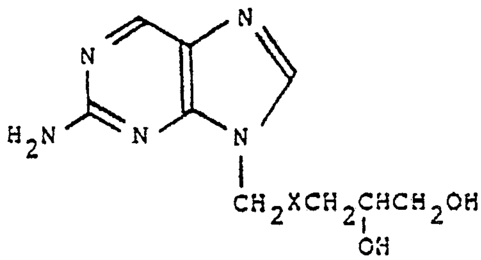

(I)

(in der X Sauerstoff oder Schwefel ist) oder ein physiologisch annehmbares Salz oder Ester davon, bei dem man :

a) eine Verbindung der Formel

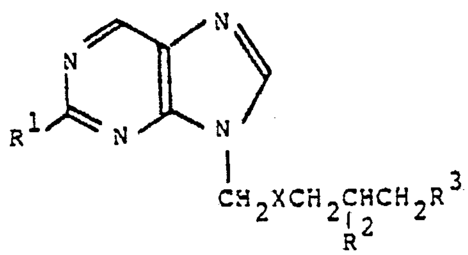

(II)

(in der X die in Anspruch 1 gegebene Bedeutung aufweist, $R^1$ eine Amino- oder blockierte Aminogruppe bedeutet, $R^2$ und $R^3$, die gleich oder verschieden sein können, jeweils eine Hydroxy- oder blockierte Hydroxygruppe bedeuten, mit der Maßgabe, daß mindestens eines von $R^1$, $R^2$ und $R^3$ eine blockierte Gruppe bedeutet, oder ein Salz oder Ester davon, entblockt ;

b) eine Verbindung der Formel

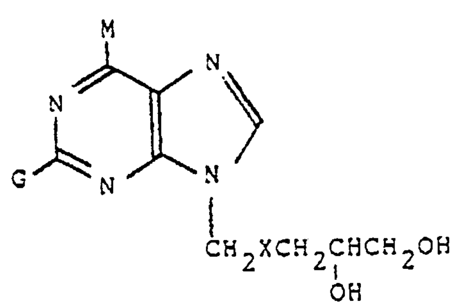

(III)

(in der X die in Anspruch 1 gegebene Bedeutung aufweist und M ein Wasserstoffatom oder einen in ein Wasserstoffatom überführbaren Rest bedeutet und G einen in eine Aminogruppe überführbaren Rest, oder (wenn M einen Rest wie vorhin definiert, bedeutet) eine Aminogruppe bedeutet, oder ein Salz oder Ester davon in eine Verbindung der Formel (I) oder ein Salz oder Ester davon überführt ;

c) eine Verbindung der Formel

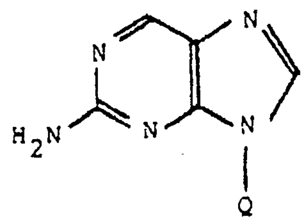

(IV)

(in der Q eine austretende Gruppe oder Atom bedeutet) mit einer Verbindung der Formel

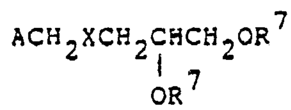

(V)

(in der X die in Anspruch 1 gegebene Bedeutung aufweist, $R^7$ eine Blockierungsgruppe ist und A eine austretende Gruppe oder Atom bedeutet), umsetzt ;

d) einen Ring in einer Vorstufenverbindung, in der entweder der Pyrimidin oder der Imidazolring unvollständig gebildet vorliegt, schließt ; und gegebenenfalls eine oder mehrere der folgenden Umwandlungen in jeder gewünschten Reihenfolge durchführt :

i) wenn das erhaltene Produkt ein Base ist, Überführung der Base in ein physiologisch annehmbares Säureadditionssalz ;

ii) wenn das erhaltene Produkt ein Säureadditionssalz ist, Überführung des Salzes in die Stammbase ;

iii) wenn das erhaltene Produkt die Verbindung der Formel (I) oder ein Salz davon ist, Überführung dieser Verbindung oder des Salzes davon in einen physiologisch annehmbaren Ester dieser Verbindung oder des Salzes, und

iv) wenn das erhaltene Produkt ein Ester der Formel (I) ist, Überführung des Esters in die Stammverbindung (I), ein physiologisch annehmbares Salz davon oder in einen verschiedenen physiologisch annehmbaren Ester.

2. Verfahren zur Herstellung einer Verbindung, wie in Anspruch 1 beansprucht, in der X Sauerstoff ist.

3. Verfahren zur Herstellung von 3-[(2-Amino-9H-purin-9-yl) methoxy]-1,2-propandiol wie in Anspruch 1 beansprucht.

**Revendications** (pour les Etats contractants : BE, CH, DE, FR, GB, IT, LI, LU, NL, SE)

1. Composés de la formule générale

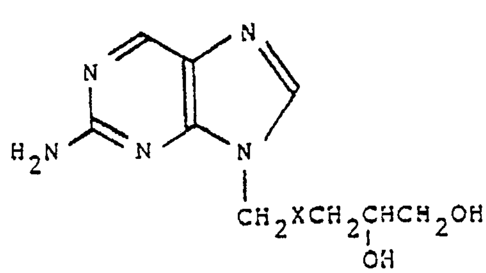

(I)

(où X est oxygène ou soufre) et leurs sels et esters physiologiquement acceptables.

2. Composés suivant la revendication 1, dans lesquels X est oxygène.

3. Le 3-[(2-amino-9H-purin-9-yl) méthoxy]-1,2-propanediol.

4. Procédé de préparation de composés de formule (I) (tels que définis dans la revendication 1) ou d'un sel ou ester physiologiquement acceptable de ceux-ci, qui comprend :

a) le déblocage d'un composé de formule

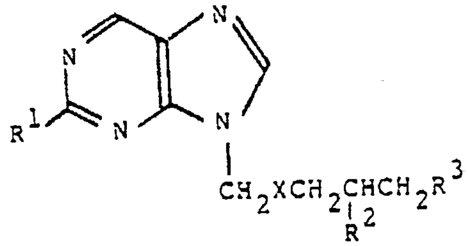

(II)

(où X est tel que défini dans la revendication 1, $R^1$ représente un radical amino ou amino bloqué, $R^2$ et $R^3$, qui peuvent être identiques ou différents, représentent chacun un radical hydroxyle ou hydroxyle bloqué, avec la restriction qu'au moins l'un d'entre $R^1$, $R^2$ et $R^3$ représente un radical bloqué) ou d'un sel ou ester de celui-ci ;

b) la conversion d'un composé de formule

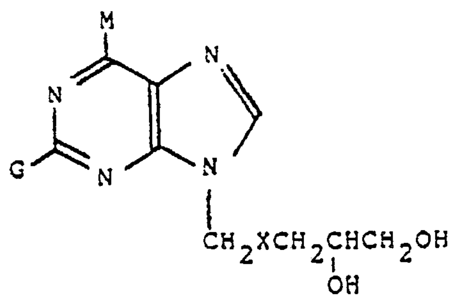

(III)

(où X est tel que défini dans la revendication 1 et M représente un atome d'hydrogène ou un radical convertible en un atome d'hydrogène et G représente un radical convertible en un radical amino ou (lorsque M représente un radical tel que défini ci-dessus) un radical amino) ou d'un sel ou ester de celui-ci en un composé de formule (I) ou en un sel ou ester de celui-ci ;

c) la réaction d'un composé de formule

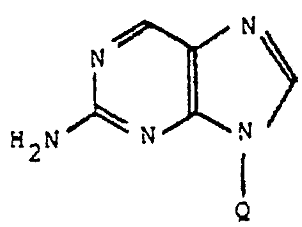

(IV)

(où Q représente un radical ou atome partant) avec un composé de formule

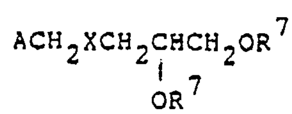

(V)

(où X est tel que défini dans la revendication 1, $R^7$ est un radical de blocage et A représente un radical ou atome partant) ;

d) la fermeture d'un cycle dans un composé précurseur dont le cycle pyrimidine ou imidazole est incomplètement formé ; et éventuellement l'exécution d'une ou plusieurs des conversions suivantes dans tout ordre souhaité :

i) lorsque le produit résultant est une base, la conversion de cette base en un sel d'addition d'acide physiologiquement acceptable de celle-ci ;

ii) lorsque le produit résultant est un sel d'addition d'acide, la conversion de ce sel en la base dont il est issu ;

iii) lorsque le produit résultant est le composé de formule (I) ou un sel de celui-ci, la conversion de ce composé ou de son sel en un ester physiologiquement acceptable de ce composé ou sel ; et

iv) lorsque le produit résultant est un ester du composé de formule (I), la conversion de cet ester en le composé de formule (I) dont il est issu, en un sel physiologiquement acceptable de celui-ci ou en un ester différent physiologiquement acceptable de celui-ci.

5. Compositions pharmaceutiques comprenant au moins un composé suivant l'une quelconque des revendications précédentes conjointement avec au moins un véhicule ou excipient pharmaceutique.

6. Composé de formule (I) (tel que défini dans la revendication 1) ou sel ou ester physiologiquement acceptable de celui-ci à utiliser en thérapeutique.

7. Composé de formule (I) (tel que défini dans la revendication 1) ou sel ou ester physiologiquement acceptable de celui-ci à utiliser pour le traitement ou la prophylaxie des infections virales.

8. Composé de formule (I) (tel que défini dans la revendication 1) ou sel ou ester physiologiquement acceptable de celui-ci à utiliser pour le traitement ou la prophylaxie des infections par herpes simplex.

**Revendications** (pour l'Etat contractant AT)

1. Procédé de préparation d'un composé de formule (I)

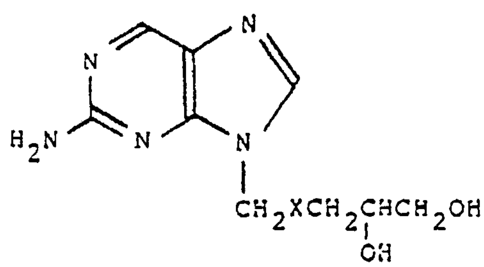

(I)

(où X est oxygène ou soufre) ou d'un sel ou ester physiologiquement acceptable de celui-ci, qui comprend

a) le déblocage d'un composé de formule

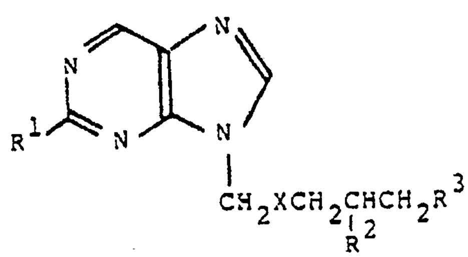

(II)

(où X est tel que défini dans la revendication 1, $R^1$ représente un radical amino ou amino bloqué, $R^2$ et $R^3$, qui peuvent être identiques ou différents, représentent chacun un radical hydroxyle ou hydroxyle bloqué, avec la restriction qu'au moins l'un d'entre $R^1$, $R^2$ et $R^3$ représente un radical bloqué) ou d'un sel ou ester de celui-ci ;

b) la conversion d'un composé de formule

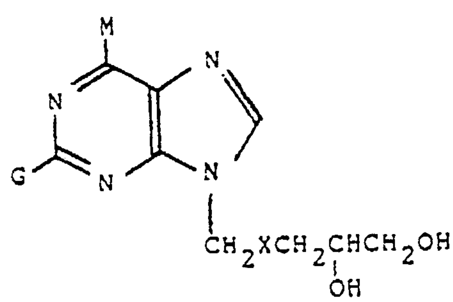

(III)

(où X est tel que défini dans la revendication 1 et M représente un atome d'hydrogène ou un radical convertible en un atome d'hydrogène et G représente un radical convertible en un radical amino ou (lorsque M représente un radical tel que défini ci-dessus) un radical amino) ou d'un sel ou ester de celui-ci en un composé de formule (I) ou en un sel ou ester de celui-ci ;

c) la réaction d'un composé de formule

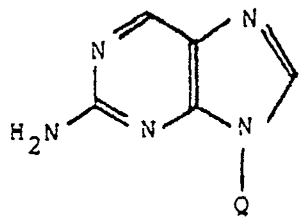

(IV)

(où Q représente un radical ou atome partant) avec un composé de formule

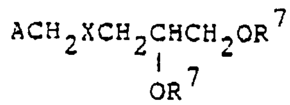

(V)

(où X est tel que défini dans la revendication 1, $R^7$ est un radical de blocage et A représente un radical ou atome partant) ;

d) la fermeture d'un cycle dans un composé précurseur dont le cycle pyrimidine ou imidazole est incomplètement formé ; et éventuellement l'exécution d'une ou plusieurs des conversions suivantes dans tout ordre souhaité :

i) lorsque le produit résultant est une base, la conversion de cette base en un sel d'addition d'acide physiologiquement acceptable de celle-ci ;

ii) lorsque le produit résultant est un sel d'addition d'acide, la conversion de ce sel en la base dont il est issu ;

iii) lorsque le produit résultant est le composé de formule (I) ou un sel de celui-ci, la conversion de ce composé ou de son sel en un ester physiologiquement acceptable de ce composé ou sel ; et

iv) lorsque le produit résultant est un ester du composé de formule (I), la conversion de cet ester en le composé de formule (I) dont il est issu, en un sel physiologiquement acceptable de celui-ci ou en un ester différent physiologiquement acceptable de celui-ci.

2. Procédé de préparation de composés suivant la revendication 1, dans lequel X est oxygène.

3. Procédé de préparation du 3-[(2-amino-9H-purin-9-yl) méthoxy]-1,2-propanediol suivant la revendication 1.